# FASCICULE DE BREVET EUROPEEN

(11) **EP 1 506 738 B1**
(45) Date de publication et mention de la délivrance du brevet: **06.05.2009**
(21) Numéro de dépôt: 04291994.4
(22) Date de dépôt: 04.08.2004
(51) Int. Cl.: A61B 5/04, A61B 5/0408, A61B 5/00

(54) **Vêtement de surveillance médicale d'un patient**
Kleidungsstück für die medizinische Patientenüberwachung
Garment for medical monitoring of a patient

(30) Priorité: 14.08.2003 FR 0309937
(43) Date de publication de la demande: 16.02.2005
(73) Titulaire: Tam Telesante, 13545 Aix en Provence Cedex 4 (FR)
(72) Inventeur: Klefstad-Sillonville, Francis, 13320 Bouc Bel Air (FR); Weber, Jean-Luc, 13300 Salon de Provence (FR); Blanc, David, 83570 Montfort sur Argens (FR)
(74) Mandataire: Ramey, Daniel

(56) Documents cités:
- WO-A-02/40091
- FR-A- 2 833 497
- US-A- 4 016 868
- US-A1- 2002 124 295
- US-B1- 6 381 482

## Description

La présente invention concerne un vêtement de surveillance médicale d'un sujet.

La surveillance médicale de personnes dans leurs activités ou situations habituelles est utile dans de nombreux cas, où le moyen de surveillance ne doit pas gêner ou perturber la vie du sujet, par exemple lors d'essais cliniques de nouveaux moyens ou méthodes thérapeutiques et également dans le cas des personnes âgées dont on souhaite suivre à distance l'état de santé dans leur milieu de vie naturel pour maintenir leur autonomie aussi longtemps que possible.

Dans d'autres applications, la surveillance et l'assistance médicales doivent être quasi-permanentes quand le patient court un danger pouvant survenir de manière aléatoire dans son activité ou sa situation et nécessitant une prompte assistance. De tels cas se présentent par exemple avec des personnes en situation risquée (pompiers, militaires en opération, personnels de maintenance dans des installations à hauts risques, sportifs de haut niveau, etc.) ou avec des nouveaux-nés risquant la mort subite du nourrisson, ou des jeunes enfants atteints de pathologies cardio-respiratoires.

La surveillance médicale quasi-continue nécessite de poser de façon durable sur le patient des capteurs biomédicaux en rapport avec la pathologie ou le risque surveillé. Par exemple, les fonctions cardio-respiratoires sont très fréquemment surveillées au moyen d'électrocardiogrammes (ECG) et de mesures respiratoires. Les capteurs correspondants peuvent être une gêne pour le patient s'ils sont volumineux ou lourds ou difficiles à emmener sur soi pendant les phases de surveillance médicale, et leurs moyens d'alimentation en énergie (des batteries en général) peuvent être lourds ou encombrants. Des solutions ont été proposées pour diminuer la gêne due aux capteurs, aux batteries ou piles et aux modules de traitement de données qui leur sont rattachés et embarqués sur le patient en fixant les capteurs et les modules sur un vêtement que le patient enfile et porte pendant ses activités surveillées (WO-A-99/64657).

Ces solutions connues ont pour inconvénient d'utiliser en général des électrodes collées sur la peau pour la saisie des électrocardiogrammes (ECG). Les électrodes collées permettent d'obtenir une bonne qualité du signal électrocardiographique, mais un temps d'installation sur le patient est nécessaire et elles provoquent des réactions dermiques et doivent être changées périodiquement. Les électrodes collées ne sont donc pas adaptées pour les patients cités plus haut, tels que les nourrissons et les personnes exposées par exemple.

On a aussi proposé d'intégrer des électrodes dans un vêtement élastique porté par le patient, mais le vêtement, souple et élastique en lui-même, est bridé par les câbles conducteurs qui le parcourent, car ces câbles ne sont pas élastiques (US -A- 6080690 et WO -A- 0102052). Il en résulte un manque de confort certain et souvent une mauvaise application des électrodes sur la peau du patient.

De plus, les câbles conducteurs et leurs connexions constituent des surépaisseurs dans le vêtement qui peuvent provoquer des irritations, en particulier sur la peau fragile des nouveaux-nés, et sur les adultes si des charges externes sont appliquées sur le corps aux endroits où passent ces câbles conducteurs.

L'alimentation électrique des capteurs pose aussi des problèmes : les piles ou batteries généralement utilisées sont à changer ou recharger périodiquement, ce qui nécessite des opérations de maintenance au cours de la surveillance médicale de longue durée, susceptibles d'interrompre cette surveillance.

Dans des dispositifs actuellement disponibles pour surveiller les nourrissons, des capteurs sont fixés à un vêtement ou collés sur la peau du bébé, et un câble électrique relie le vêtement porté par le bébé à un boîtier extérieur proche qui fournit l'énergie et recueille les données des capteurs.

Le document US 6,381,482 décrit un système tel que défini dans le préambule de la revendication 1.

Ce câble de liaison constitue une gêne et un danger pour le bébé.

Pour résoudre ces problèmes et éviter les inconvénients précités, l'invention propose un vêtement de surveillance médicale d'un patient, comprenant des capteurs biomédicaux, des moyens de distribution de l'énergie électrique et de transmission des données, et des moyens d'alimentation électrique et de transfert des données vers des moyens extérieurs au vêtement, au moins certains des capteurs, des moyens de distribution ou d'alimentation électrique, et des moyens de transmission ou de transfert des données étant formés de fils conducteurs intégrés et répartis dans le tissu du vêtement, caractérisé en ce que les fils conducteurs sont constitués de fils conducteurs fins enroulés en spirale autour d'une âme élastique non conductrice et sont élastiques.

Ce vêtement a l'avantage d'imiter la peau du corps humain et de reproduire sur la partie du corps concernée une enveloppe qui a des propriétés de souplesse et d'élasticité, qui contient des capteurs intégrés souples et élastiques, et qui fait circuler les signaux émis par les capteurs sur des réseaux électriques dont on maîtrise la conductivité, c'est à dire un vêtement qui se comporte comme « une seconde peau ».

Le vêtement est utilisable dans un système de surveillance médicale qui optimise les compromis entre la qualité des signaux physiologiques recueillis, le confort et l'autonomie électrique, et qui est applicable en particulier aux personnes exposées (professionnels en situation risquée ou sportifs de haut niveau par exemple), aux nouveaux-nés et jeunes enfants, aux essais cliniques et aux personnes âgées.

L'énergie électrique est apportée à l'électronique du vêtement par un transfert extérieur sans contact. Le système de surveillance associé au vêtement comprend un moyen pour recueillir et traiter localement les données venant des capteurs, transférées sans contact depuis le vêtement, et un serveur distant auquel du personnel médical peut se connecter pour consulter et interpréter les données des capteurs reçues via un réseau de télécommunication ou de transmission de données.

Avantageusement, le vêtement ou plus généralement une enveloppe d'une partie du corps humain, qui est porté à même la peau, a toutes les caractéristiques de protection mécanique, de confort, d'évacuation de l'humidité et d'isolation thermique d'un bon sous-vêtement souple et élastique qui épouse les formes du corps tout en comprenant des capteurs physiologiques et des fils conducteurs souples et élastiques intégrés, les capteurs physiologiques (par exemple des électrodes pour ECG ou des capteurs acoustiques) étant appliqués contre la peau par les effets de leur élasticité et de celle du vêtement, sans nécessiter de collage.

L'invention sera mieux comprise à la lecture de la description qui suit, faite à titre d'exemple non limitatif en référence aux dessins annexés dans lesquels :
- La figure 1 est une vue schématique d'un sous-vêtement intégrant des fils conducteurs et des capteurs ;
- La figure 2 est une vue agrandie d'un fil conducteur rendu élastique par enroulement autour d'une âme élastique (procédé de guipage) ;
- Les figures 3 et 4 représentent des liaisons conductrices entre deux fils conducteurs élastiques ;
- La figure 5 représente une variante de réalisation de la liaison ;
- La figure 6 montre une liaison conductrice entre un fil conducteur élastique et un composant non élastique ;
- Les figures 7 et 8 représentent un exemple de capteur souple tissé dans le tissu du vêtement ;
- La figure 9 est une vue schématique en coupe d'un tissu réalisé en plusieurs couches tissées ou tricotées ;
- La figure 10 représente un nouveau-né habillé d'un vêtement selon l'invention ;
- La figure 11 est une vue partielle montrant la position des enroulements de couplage permettant des transferts sans contact entre les circuits dans le vêtement porté par un bébé et la housse du matelas sur lequel il dort ;
- La figure 12 montre une installation de surveillance du sommeil du nourrisson.

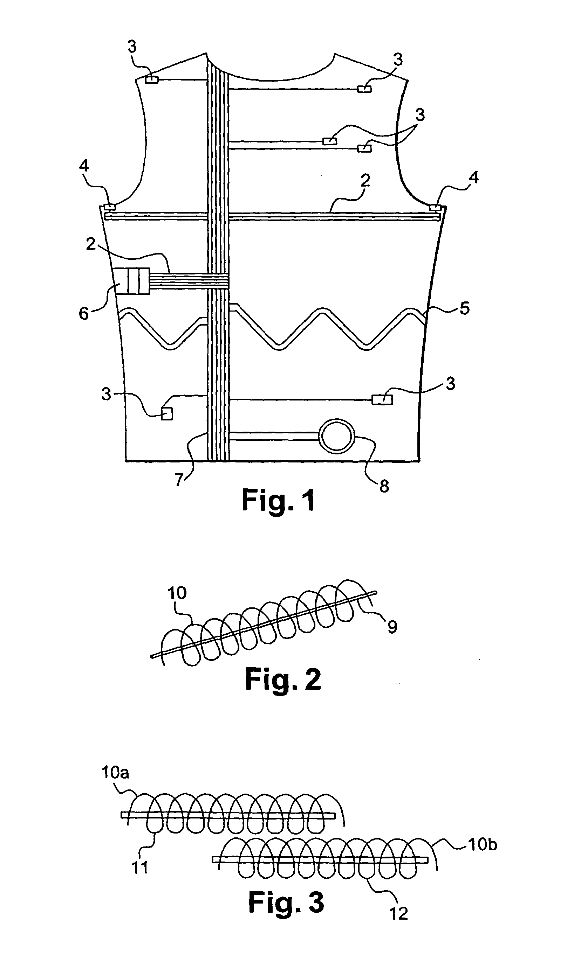

La figure 1 est une vue de face d'un sous-vêtement 1 (représenté sans manches) en tissu dans lequel sont intégrés des fils conducteurs isolés électriquement, dont certains sont regroupés en bandes ou nappes 2 et 7. Le vêtement 1 comprend ici un bus 7 de transmission de données et d'alimentation électrique d'un type classique en électronique (tel que par exemple le « bus de terrain » 12C), relié à une boucle 8 qui est un enroulement de couplage inductif permettant de transmettre l'énergie et /ou les données à un enroulement voisin (non représenté et ne faisant pas partie du vêtement) qui n'est pas en contact direct avec le vêtement.

Les capteurs intégrés dans le vêtement comprennent, par exemple, des électrodes ECG 3, des sondes de température 4, un moyen 5 de mesure de variation du périmètre thoracique et un capteur d'activité ou de position 6.

Le sous-vêtement est réalisé en tissu ou tricot de fils élastiques non conducteurs, par exemple du type commercialisé sous la marque « Elasthanne » par Dupont de Nemours, qui assurent une grande souplesse et une grande élasticité, comme dans les maillots couramment réalisés pour les sportifs, la totalité du vêtement ou au moins une majeure partie de celui-ci étant formée de ces fils élastiques non conducteurs.

Les fils conducteurs tels que 2 et 7 intégrés dans le vêtement ne restreignent pas l'élasticité ni le confort du vêtement, car ils sont eux-mêmes élastiques. Un avantage substantiel de l'invention qui résulte de la grande élasticité du vêtement, est de limiter le nombre de tailles différentes à mettre sur le marché pour satisfaire les différents morphotypes des utilisateurs.

Toutes les caractéristiques décrites ici pour un sous-vêtement s'appliquent à toute enveloppe souple et élastique d'une partie du corps, comme par exemple un tour de cou, une chaussette, une manche, un gant, etc.

La figure 2 montre comment, selon l'invention, un fil conducteur non extensible au départ (fil métallique ou fil en polymère conducteur ou fil en polymère chargé ou revêtu d'un matériau conducteur par exemple) est rendu élastique : le fil conducteur 10 est enroulé en spirale autour d'une âme constituée par un fil élastique 9 non conducteur qui est d'une matière telle qu'après avoir été soumis à une traction, il revient à sa longueur initiale, et qui est par exemple en « Elasthanne » ou en une matière analogue.

Cette opération d'enroulement appelée guipage est réalisable par des machines classiques dans des ateliers de filature. L'effet mécanique de la combinaison fil élastique 9 - fil conducteur 10 en spirale est double : lors d'une traction, l'ensemble s'allonge dans le sens de la traction en opposant une réaction comme un ressort (l'âme élastique 9 et la spirale 10 résistent ensemble dans le même sens), et lorsque la traction cesse, l'ensemble est ramené à sa longueur initiale par l'âme élastique 9 qui comprime la spirale conductrice 10. Il est possible d'enrouler autour de cet ensemble un autre fil textile. Cette opération dite de guipage inverse ne nuit pas à l'élasticité de l'ensemble résultant et a pour but d'améliorer sa tenue et de faciliter son tissage.

En utilisant une âme élastique 9 ayant des propriétés mécaniques et un diamètre comparables à ceux des autres fils élastiques utilisés dans le tissu du vêtement, et un fil conducteur 10 souple et fin d'un diamètre de quelques dizaines de micromètres (typiquement 20 à 40 µm) ayant ou non un revêtement électriquement isolant selon l'usage du fil, le fil conducteur élastique obtenu par guipage se comporte au tissage ou au tricotage comme les autres fils élastiques. Le tissu résultant, dans lequel les fils conducteurs élastiques sont tissés ou tricotés, se comporte de manière homogène, sans points ni zones dures ou non élastiques. Le tissu est nettoyable et lavable avec les mêmes facilités et contraintes que s'il ne contenait pas de fils conducteurs. Le vêtement confectionné avec ce tissu a toutes les propriétés de souplesse et d'élasticité d'un vêtement qui n'aurait pas de fils conducteurs intégrés.

Selon l'usage du fil conducteur dans le vêtement, le fil est nu (le contact avec sa surface est alors conducteur) ou pourvu d'un revêtement électriquement isolant.

Par exemple, les électrodes conductrices appliquées contre la peau sont réalisées avec des fils nus. De telles électrodes peuvent servir à recueillir des signaux électriques engendrés par le corps pour l'élaboration d'un électrocardiogramme ou d'un électromyogramme par exemple), ou à transmettre au corps de l'énergie ou des impulsions. De même un capteur de déformation mécanique est réalisé avec des fils nus dont les contacts électriques entre les spires élastiques voisines 10a et 10b (figure 3) varient avec l'allongement qui leur est imposé, entraînant une variation de résistance électrique mesurable qui est une fonction de l'allongement. Un tel capteur élastique intégré dans le vêtement est utile pour mesurer par exemple les variations du périmètre thoracique du patient ou les déformations des muscles et des articulations.

Les fils conducteurs isolés électriquement sont utilisés par exemple pour transmettre les signaux et l'énergie électrique dans le vêtement.

Les fils conducteurs élastiques intégrés dans le tissu doivent pouvoir être reliés électriquement entre eux et à des composants électroniques (des capteurs et des connecteurs par exemple) intégrés dans le vêtement.

Il est connu que les bonnes liaisons électriques fixes sont faites par brasure entre deux conducteurs, mais aussi que les conducteurs cassent à proximité des brasures lorsqu'ils sont souples et soumis à des efforts mécaniques. Or dans le vêtement selon l'invention, les fils conducteurs sont soumis à de fréquents efforts mécaniques en raison de leur élasticité. L'invention évite ces problèmes dus aux brasures entre conducteurs en utilisant uniquement les propriétés mécaniques des fils conducteurs élastiques enroulés en spirale pour les relier électriquement.

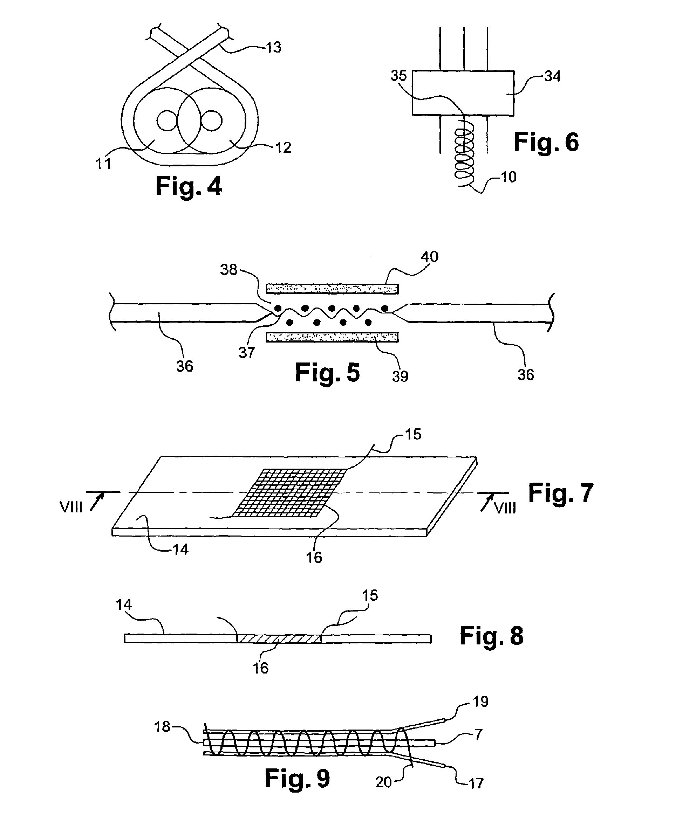

Les figures 3 et 4 illustrent un procédé selon l'invention de liaison électrique entre deux fils conducteurs élastiques 11 et 12. Tout d'abord, si les fils sont isolés électriquement, l'isolant électrique qui recouvre chaque fil conducteur 10a et 10b des fils composites 11 et 12 est éliminé sur une courte longueur de chaque fil composite. Pour cela différents procédés sont applicables, par exemple l'action chimique d'un produit qui dissout ou détruit cet isolant, ou l'action mécanique abrasive d'un jet d'air contenant des particules abrasives, ou le chauffage par une source ponctuelle de chaleur (par exemple un faisceau laser focalisé sur la zone à dénuder). Puis les portions dénudées des deux fils composites sont rapprochées (figure 3) et maintenues fermement et définitivement l'une contre l'autre par des points de ligature serrés réalisés au moyen d'un fil 13 (figure 4), qui peut être lui-même conducteur, à l'aide d'une machine à coudre. La figure 4 montre la ligature avant le serrage du fil 13 pour plus de clarté, car après serrage, les différents fils sont écrasés les uns sur les autres.

Dans une variante illustrée à la figure 5, la ligature entre deux fils ou deux bandes de fils conducteurs 37 et 38 (par exemple les fils 37 sont en trame et les fils 38 sont en chaîne dans le tissu 36) est assurée en appliquant fortement et éventuellement en chauffant, une petite pastille 39, 40 sur chaque côté de la connexion à réaliser. Les deux pastilles dont l'une au moins est électriquement conductrice, sont maintenues solidaires l'une contre l'autre en serrant et écrasant les fils à connecter 37 et 38, par exemple lorsqu'elles sont en matériaux adhésifs conducteurs, ou contiennent de la brasure pour connexion électrique qui a été chauffée et a imprégné les fils conducteurs, ou sont cousues l'une à l'autre. Ce mode de connexion convient également pour lier électriquement des fils dans un tricot ou encore pour connecter des fils étrangers au tissu à ceux du tissu. Dans ce cas, les pastilles enserrent en même temps les fils du tissu et les fils étrangers.

Ces pastilles peuvent être en métal conducteur, par exemple en cuivre ou en matériau souple tel que du kapton, avec un dépôt de brasure ou analogue. On peut aussi utiliser, non des pastilles pleines, mais de petites grilles dont les fils comportent de la brasure ou analogue.

Enfin, on dépose une matière électriquement isolante (par exemple un vernis isolant souple ou de la bande adhésive souple) sur la liaison ainsi réalisée pour reconstituer si nécessaire l'isolant électrique. On obtient ainsi une liaison sans point dur mécanique, dont les propriétés mécaniques, en particulier l'élasticité, sont homogènes avec celles des autres fils (conducteurs ou non) du tissu.

La figure 6 montre comment une liaison électrique entre un fil conducteur élastique 10 et un composant électronique 34 non élastique peut être réalisée en utilisant aussi les propriétés mécaniques de la spirale de fil conducteur : cette spirale est forcée pour venir coiffer la broche 35 du composant à relier, sur une longueur suffisante pour assurer un bon contact électrique, puis le contact est renforcé par exemple par une colle conductrice et enfin une couche isolante est déposée sur la liaison. On a réalisé ainsi un genre de sertissage mécanique. Le composant non élastique peut être souple en lui-même, s'il est réalisé par exemple sur un support mince déformable par flexion ou torsion, tel que le « Kapton » commercialisé par Dupont de Nemours.

Pour repérer facilement les fils conducteurs au milieu des fils non conducteurs et vérifier les dénudages locaux, un marquage lumineux peut être réalisé, selon l'invention en incorporant des pigments dans la couche extérieure isolante des fils conducteurs, ces pigments étant non visibles en lumière naturelle, et visibles avec certains éclairages spéciaux, par exemple ultraviolets.

Les procédés de liaison décrits ci-dessus peuvent être facilement automatisés et permettent de réaliser les liaisons électriques des fils conducteurs dans le tissu, avant ou après la confection du vêtement. Par exemple, c'est ainsi que les fils conducteurs des nappes 2 (figure 1) qui vont aux capteurs 3, 4, 5 et 6 sont reliés aux fils conducteurs de la nappe 7 pour former le bus électronique du vêtement.

Les figures 7 et 8 montrent comment les capteurs souples sont intégrés dans le tissu du vêtement. L'exemple illustré est une électrode pour ECG, constituée par crochetage dans le tissu 14 d'un fil conducteur souple et fin 15 non isolé, pour former une pièce 16 d'environ 2 X 2 cm² de surface. L'intégration de cette pièce 16 est réalisable sur des métiers à tisser du type Jacquard avec la technique connue dite des bâtons brocheurs. Le fil dont est faite l'électrode 16 peut être par exemple en acier inoxydable, en cuivre chemisé d'argent ou d'or, ou en or massif ou en polymères conducteurs. La liaison d'un tel capteur avec les fils conducteurs élastiques du bus 7, est réalisable par le procédé de rapprochement de portions dénudées resserrées par ligatures exposé plus haut.

Selon une variante de l'invention, les électrodes ou les parties conductrices du vêtement ne sont pas intégrées dans le tissu, mais sont le tissu lui-même. Par exemple, le tissu est totalement réalisé avec des fils conducteurs en polymère isolant revêtu de métal conducteur. Les zones où la conduction des fils n'est pas souhaitée (par exemple autour d'une électrode) sont traitées chimiquement ou électro-chimiquement (par exemple par électrolyse) pour enlever le revêtement de métal sur les fils polymères, ce qui rend la zone non conductrice en dehors de la partie conductrice qu'on a préservée. Inversement, une zone non conductrice du tissu peut être rendue conductrice par les mêmes procédés chimiques ou électro-chimiques ou par imprégnation ou projection de matière conductrice sur la zone à traiter. Ces procédés permettent de faire varier à volonté la conductivité du tissu, même après le tissage, ce qui procure une grande souplesse dans la conception et l'adaptation du vêtement à ses conditions d'utilisation.

La figure 9 est une vue en coupe d'un tissu pour le vêtement selon l'invention, constitué de plusieurs couches rendues solidaires les unes les autres, dans le but d'assurer en particulier la protection électromagnétique du vêtement.

Dans l'usage du vêtement avec capteurs et fils conducteurs intégrés, les fils conducteurs répartis dans le vêtement peuvent jouer le rôle d'antenne vis à vis des phénomènes électromagnétiques gênants reçus de l'extérieur et rayonnés de l'intérieur. Le remède courant contre ces perturbations est le blindage des fils par des couches conductrices qui entourent les fils qu'on veut protéger. Selon l'invention, le procédé de blindage électromagnétique du vêtement consiste à insérer la couche 18 (figure 9) dans laquelle sont les nappes de conducteurs à protéger telles que par exemple 2 et 7 (figure 1) entre une nappe de fils conducteurs isolés tissés dans une couche de tissu 17 par dessous et une autre nappe de fils conducteurs isolés tissés dans une couche 19 au dessus. Les nappes conductrices des couches 17 et 19 sont reliées électriquement entre elles en certains points dans le vêtement, et reliées à un niveau de potentiel électrique généralement appelée « la masse ». De plus, chaque couche peut jouer un rôle précis différencié dans le confort du vêtement comme connu pour les tissus à plusieurs couches.

Les différentes couches qui forment le tissu complet sont solidarisées entre elles au moyen de fils 20 par un procédé de tissage ou de couture habituel.

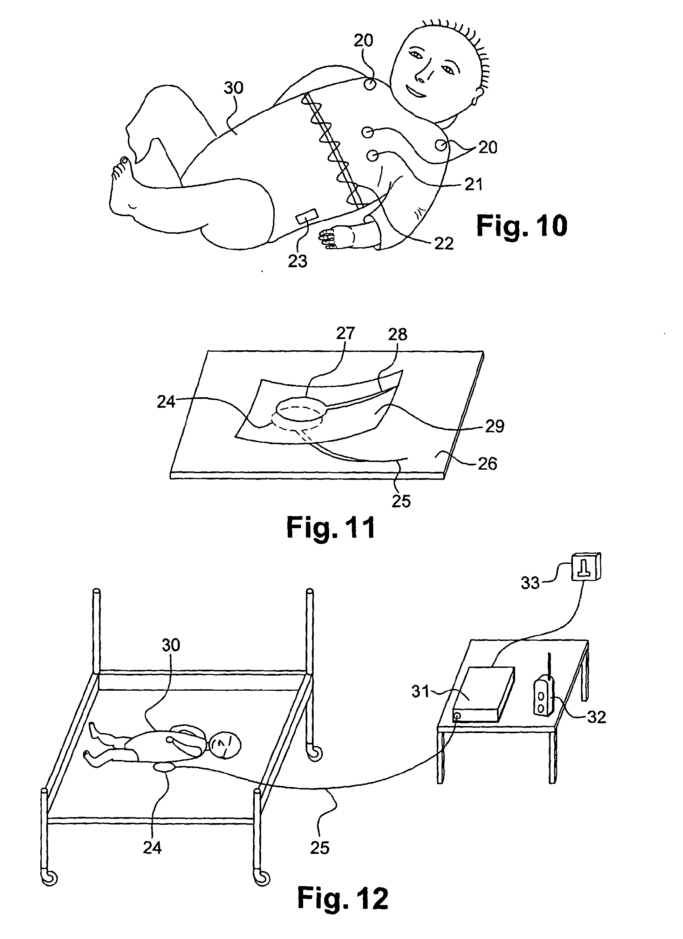

La figure 10 montre un nourrisson portant un vêtement 30 selon l'invention, plus particulièrement adapté à la surveillance de l'apnée du nourrisson. Pour simplifier le dessin, les fils conducteurs ne sont pas figurés, seuls les capteurs intégrés dans le vêtement ont été représentés. Les paramètres adaptés à la surveillance de l'apnée du nourrisson, en vue de prévenir son éventuelle mort subite, sont de préférence : la fréquence cardiaque, obtenue par exemple à partir d'électrodes textiles souples 20 appliquées contre la peau, ou à partir d'un balistomètre (capteur électroacoustique) 21 posé à proximité du coeur, et la fréquence respiratoire, obtenue par exemple par la saisie des mouvements respiratoires par un pléthysmographe 22 qui entoure le bas de la cage thoracique de l'enfant. On a constaté que le nombre des morts subites des nourrissons diminue si on impose aux nourrissons de dormir sur le dos. A partir de l'âge de quelques mois, les bébés posés sur le dos pour dormir ont tendance à chercher à se retourner et donc à se mettre inconsciemment dans une position plus dangereuse. Il est important que l'entourage du bébé soit informé du changement de position du bébé sur son lit. Avantageusement, le capteur de position 23 intégré dans le vêtement, détecte si l'enfant est sur le dos ou ne l'est pas ; les signaux de ce capteur sont transmis et analysés comme ceux des autres capteurs.

Afin de ne pas restreindre le confort et la facilité d'utilisation du vêtement, il importe de limiter les cartes électroniques et les sources d'énergie à installer sur le vêtement. L'invention propose pour cela une transmission de l'énergie électrique et des données entre le vêtement et son environnement physique proche, par des procédés ne nécessitant pas de contact physique avec le vêtement. Dans la présente invention, un circuit appelé secondaire est dans le vêtement et va recevoir l'énergie d'un autre circuit appelé primaire, qui est relié à une source d'énergie électrique extérieure au vêtement et est traversé par un courant alternatif de basse tension (par exemple de 6 à 24 volts).

Chaque circuit comprend un enroulement de nombreuses spires. L'enroulement du circuit primaire crée un champ d'induction qui est capté par l'enroulement du circuit secondaire placé à proximité et ainsi une énergie suffisante pour faire fonctionner les capteurs est transférée de la source d'énergie extérieure au circuit dans le vêtement. Le rendement de ce procédé dit de couplage inductif peut être augmenté en mettant des noyaux ferromagnétiques au milieu des enroulements. L'enroulement du circuit secondaire dans le vêtement est réalisable avec les fils conducteurs élastiques selon l'invention, pour ne pas limiter la souplesse et l'élasticité du vêtement à cet endroit, par exemple avec un métier à tisser Jacquard avec bâtons brocheurs. Le noyau ferromagnétique de l'enroulement secondaire souple peut être souple lui-même, en utilisant par exemple des supports en mousse synthétique souple chargée de particules ferromagnétiques.
La figure 11 montre un exemple de couplage inductif entre un enroulement 24 (en traits pointillés) du circuit primaire 25 intégré dans le drap 26 ou la housse du matelas du lit du nourrisson et un enroulement 27 du circuit secondaire 28 intégré dans le dos du vêtement 29 du bébé (dont n'est représentée qu'une faible surface) qui reste proche de l'enroulement primaire 24, puisque le bébé a été posé sur le dos contre le drap.

En cas de dysfonctionnement du couplage inductif dû par exemple à un écartement excessif des enroulements 24 et 27, et pour sécuriser le fonctionnement du système, l'énergie nécessaire au fonctionnement du vêtement peut être fournie par des piles ou batteries de secours ou encore par des « supercapacités » (condensateurs de grande capacité sous un faible volume) en attendant par exemple que les enroulements soient à nouveau rapprochés par une assistance extérieure alertée par le moyen d'alerte. Les piles peuvent être montées dans le vêtement à un endroit peu gênant, comme l'arrière du vêtement pour le bébé, au droit de l'épaisseur molle constituée par les couches-culottes.

Le mode de couplage inductif s'applique aux vêtements pour adultes, les enroulements primaire et secondaire étant maintenus proches l'un de l'autre par exemple au niveau de la ceinture d'un pantalon ou d'une jupe, ou à tout autre endroit où les mouvements relatifs de l'enroulement primaire par rapport à l'enroulement secondaire dans le vêtement, sont limités.

Le transfert sans contact des données fournies par les capteurs du vêtement à une base de recueil proche peut être réalisé également par couplage inductif. Des variantes de procédés de transfert utilisent des moyens d'échange de données sans fils disponibles dans l'industrie, par exemple par radio de courte portée (type « Bluetooth » ou autre), par infrarouge ou encore par ultrasons.

Le système de surveillance médicale à distance associé au vêtement comprend des modules extérieurs au vêtement, notamment un module de fourniture d'énergie au vêtement par couplage inductif, un module qui recueille les données des capteurs du vêtement, un module de traitement local des données, un module d'alerte de l'entourage du patient pour lui porter assistance, et un module de transmission des données à une station distante (serveur). Tous ou certains de ces modules peuvent être regroupés dans un même boîtier, appelé base de recueil proche et situé à proximité du vêtement en utilisation. Dans les applications de l'invention pour les adultes, la base de recueil proche peut être installée par exemple dans un étui ou une poche sur le porteur du vêtement. Dans les applications pour les nouveaux nés et les jeunes enfants, la base de recueil peut être posée sur le lit ou à côté du lit de l'enfant.

Le module d'alerte de l'entourage du bébé peut être avantageusement connecté à un transmetteur du commerce qui permet aux parents d'écouter à distance, par radio de courte portée, les bruits émis par le bébé dans une pièce voisine.

Le module de transmission de données à une station distante comprend en général un modem connecté à un réseau d'échange de données tel que le réseau téléphonique ou un réseau de radiocommunication (réseau GSM par exemple), ou un moyen de radiocommunication spécifique pour les personnes exposées (militaires, pompiers, sportifs...)

Quand le porteur du vêtement est en situation ambulatoire, le système de surveillance médicale peut être avantageusement complété par un moyen de localisation du porteur du vêtement, tel que le « Global Positioning System » (GPS) ou le système de géolocalisation des opérateurs de réseaux de téléphonie mobile GSM. La localisation est transmise à la station distante de la même manière que les données provenant du vêtement.

La figure 12 représente un exemple d'installation complète d'un système pour la surveillance du nourrisson dans son lit. Le bébé porte un vêtement 30 selon l'invention, tel que par exemple celui de la figure 9. L'enroulement 24 de couplage inductif du circuit d'énergie primaire 25 est seul représenté, pour la clarté du dessin. L'enroulement 24 est intégré dans la housse du matelas, d'où sort le câble 25 du circuit primaire, connecté à la base de recueil proche 31 disposée à coté du lit. Le transfert de données des capteurs du vêtement vers la base de recueil 31, est assuré lui aussi par couplage inductif, mais les circuits correspondants ne sont pas figurés. La base 31 contient des batteries qui fournissent l'énergie primaire. Les moyens de traitement local et de déclenchement d'une alerte sont contenus dans la base de recueil ; le moyen de déclenchement est connecté à un dispositif radio de courte portée 32 fonctionnant sur piles ou sur batteries du type de ceux qui permettent aux parents d'écouter les bruits faits par l'enfant dans une pièce voisine. Ce dispositif radio constitue dans cet exemple le moyen d'alerter l'entourage proche du bébé. La base proche 31 contient aussi un modem pour transmettre les données des capteurs à un serveur distant via le réseau téléphonique filaire dans cet exemple, représenté ici par une prise téléphonique 33. Une telle installation du système procure à l'enfant une double isolation électrique par rapport à l'installation électrique du logement. Les données télétransmises au serveur sont examinées et interprétées par le médecin traitant et un expert d'une manière connue en soi (FR -A- 2717332 et FR -A- 2760962).

## Revendications

1. Vêtement de surveillance médicale d'un patient, comprenant des capteurs biomédicaux (3,4,5,6), des moyens (2,7) de distribution de l'énergie électrique et de transmission des données, et des moyens (8) d'alimentation électrique et de transfert des données vers des moyens (31) extérieurs au vêtement, au moins certains des capteurs (3,4,5,6), des moyens (2,7) de distribution ou d'alimentation électrique, et des moyens (8) de transmission ou de transfert des données étant formés due fils conducteurs (11,12) intégrés et répartis dans le tissu du vêtement, **caractérisé en ce que** les fils conducteurs (11,12) sont constitués de fils conducteurs fins (10) enroulés en spirale autour d'une âme élastique (9) non conductrice et sont élastiques.

2. Vêtement selon la revendication 1, **caractérisé en ce que** des capteurs (16) constitués de fils conducteurs élastiques sont intégrés dans le tissu du vêtement au cours du tissage du vêtement.

3. Vêtement selon la revendication 1 ou 2, **caractérisé en ce qu'**une liaison conductrice entre deux fils conducteurs élastiques (11,12) est réalisée par contact des spirales (10a et 10b) des deux fils conducteurs élastiques, le contact étant maintenu par des points de ligature (13) serrant les deux fils conducteurs l'un contre l'autre.

4. Vêtement selon l'une des revendications 1 ou 2, **caractérisé en ce qu'**une liaison conductrice entre deux fils conducteurs (37,38) est réalisée en serrant les fils l'un contre l'autre entre deux pastilles ou grilles (39,40) rendues solidaires l'une de l'autre et dont l'une au moins est conductrice.

5. Vêtement selon l'une des revendications 1 à 4, **caractérisé en ce qu'**un au moins des capteurs mesure des déformations mécaniques de la partie du corps sur lequel il est appliqué, en enregistrant des effets électriques dus au glissement entre elles des spirales des fils conducteurs élastiques (10a, 10b) lorsque le tissu est sollicité en traction dans le sens des fils conducteurs.

6. Vêtement selon l'une des revendications 1 à 5, **caractérisé en ce que** des zones conductrices ou des zones non conductrices sont créées en modifiant sur le vêtement ou sur le tissu la conductivité des fils conducteurs par traitement chimique ou électro-chimique ou par imprégnation de matière conductrice ou isolante.

7. Vêtement selon l'une des revendications 1 à 6, **caractérisé en ce que** le vêtement est constitué de plusieurs couches de tissu superposées (17,18,19) pouvant contenir des fils conducteurs, en particulier pour protéger les circuits du vêtement contre des effets électromagnétiques indésirable, et maintenues les unes contre les autres par des fils (20) les traversant.

8. Vêtement selon l'une des revendications 1 à 7, **caractérisé en ce que** l'énergie électrique et/ou les données des capteurs sont transportées dans le vêtement par un bus de fils conducteurs élastiques isolés (7) auquel les capteurs sont reliés.

9. Vêtement selon l'une des revendications 1 à 8, **caractérisé en ce que** l'énergie électrique nécessaire au fonctionnement des capteurs (3,4,5,6) dans le vêtement est fournie par couplage inductif sans contact entre une source d'énergie extérieure et un circuit spécifique (8) intégré dans le vêtement.

10. Vêtement selon l'une des revendications 1 à 9, **caractérisé en ce que** le transfert des données saisies par les capteurs du vêtement, vers une base de recueil proche (31), est réalisé par une liaison sans contact, telle qu'une liaison radio de courte portée ou infrarouge, ou par couplage inductif.

11. Vêtement selon l'une des revendications 1 à 10, **caractérisé en ce que** les données fournies par les capteurs sont transmissibles, via un réseau de télécommunications, à un serveur utilisable par du personnel médical.

## Claims

1. Garment for the medical monitoring of a patient, comprising biomedical sensors (3, 4, 5, 6), electrical power distribution and data transmission means (2, 7) and means (8) for electrical power and for data transfer to means (31) external to the garment, at least some of the sensors (3, 4, 5, 6), the electrical supply or distribution means (2, 7) and the data transmission or transfer means (8) being formed from conducting yarns (11, 12) integrated into and distributed over the fabric of the garment, **characterized in that** the conducting yarns (11, 12) consist of fine conducting yarns (10) wound in a spiral around a non-conducting elastic core (9) and are elastic.

2. Garment according to Claim 1, **characterized in that** sensors (16) formed from elastic conducting yarns are integrated into the fabric of the garment while the garment is being woven.

3. Garment according to Claim 1 or 2, **characterized in that** a conducting connection between two elastic conducting yarns (11, 12) is produced by contact between the spirals (10a and 10b) of the two elastic conducting yarns, the contact being maintained by binding stitches (13) that clamp the two conducting yarns against each other.

4. Garment according to Claim 1 or 2, **characterized in that** a conducting connection between two conducting yarns (37, 38) is produced by clamping the yarns against each other between two pads or meshes (39, 40) fastened together, at least one of which is conducting.

5. Garment according to one of Claims 1 to 4, **characterized in that** at least one of the sensors measures mechanical deformations of that part of the body on which it is applied, by recording electrical effects due to the mutual sliding of the spirals of the elastic conducting yarns (10a, 10b) when the fabric is pulled in the direction of the conducting yarns.

6. Garment according to one of Claims 1 to 5, **characterized in that** the conducting areas or non-conducting areas are created by modifying, on the garment or on the fabric, the conductivity of the conducting yarns by chemical or electrochemical treatment or by impregnation with a conductive or insulating material.

7. Garment according to one of Claims 1 to 6, **characterized in that** the garment consists of several superposed fabric layers (17, 18, 19) that may contain conducting yarns, in particular for protecting the circuits of the garment from undesirable electromagnetic effects, the said layers being held in place against one another by yarns (20) that pass through them.

8. Garment according to one of Claims 1 to 7, **characterized in that** the electrical power and/or the data from the sensors are transported within the garment by a bus (7) of insulated elastic conducting yarns, to which the sensors are connected.

9. Garment according to one of Claims 1 to 8, **characterized in that** the electrical power needed to operate the sensors (3, 4, 5, 6) in the garment is supplied by contactless inductive coupling between an external power supply and a specific circuit (8) integrated into the garment.

10. Garment according to one of Claims 1 to 9, **characterized in that** the data picked up by the sensors in the garment is transferred to a close receiver base (31) via a contactless link, such as a short-range radio link or infrared link, or by inductive coupling.

11. Garment according to one of Claims 1 to 10, **characterized in that** the data delivered by the sensors can be transmitted, via a telecommunications network, to a server that can be used by medical personnel.

## Patentansprüche

1. Kleidungsstück für die medizinische Überwachung eines Patienten, umfassend biomedizinische Sensoren (3, 4, 5, 6), Mittel (2, 7) zur Verteilung elektrischer Energie und zur Übertragung von Daten, und Mittel (8) für die elektrische Zuführung und den Transport von Daten an Mittel (31) außerhalb des Kleidungsstückes, wobei wenigstens bestimmte der Sensoren (3, 4, 5, 6), der Mittel (2, 7) zur Verteilung oder Einspeisung von Strom und der Mittel (8) für die Übertragung oder den Transport von Daten aus Leitungsdrähten (11, 12) gebildet sind, welche in dem Gewebe des Kleidungsstückes integriert und verteilt sind, **dadurch gekennzeichnet, dass** die Leitungsdrähte (11, 12) aus feinen Leitungsdrähten (10) bestehen, welche um einen elastischen Kern (9), der nicht-leitend ist, spiralenförmig aufgewickelt sind und elastisch sind.

2. Kleidungsstück gemäß Anspruch 1, **dadurch gekennzeichnet, dass** die Sensoren (16), bestehend aus elastischen Leitungsdrähten, in das Gewebe des Kleidungsstückes während des Webens des Kleidungsstückes integriert werden.

3. Kleidungsstück gemäß Anspruch 1 oder 2, **dadurch gekennzeichnet, dass** eine leitende Verbindung zwischen zwei elastischen Leitungsdrähten (11, 12) durch Kontakt von Spiralen (10a und 10b) von zwei elastischen Leitungsdrähten realisiert ist, wobei der Kontakt durch Ligaturpunkte (13), welche die beiden Leitungsdrähte einen gegen den anderen klemmen, aufrechterhalten wird.

4. Kleidungsstück gemäß den Ansprüchen 1 oder 2, **dadurch gekennzeichnet, dass** eine elektrische Verbindung zwischen zwei Leitungsdrähten (37, 38) realisiert ist, indem die Drähte gegeneinander zwischen zwei Plättchen oder Scheiben (39, 40) geklemmt werden, die miteinander haftend gemacht sind und wovon wenigstens eines leitend ist.

5. Kleidungsstück gemäß den Ansprüchen 1 bis 4, **dadurch gekennzeichnet, dass** einer wenigstens der Sensoren mechanische Verformungen der Körperpartie misst, auf welcher er aufgebracht ist, indem elektrische Effekte infolge von Verschiebungen zwischen den Spiralen der elastischen Leitungsdrähte (10a, 10b) registriert werden, wenn das Gewebe in Richtung der Leitungsdrähte zugbeansprucht wird.

6. Kleidungsstück gemäß den Ansprüchen 1 bis 5, **dadurch gekennzeichnet, dass** leitende Bereiche oder nicht-leitende Bereiche geschaffen sind, indem in dem Kleidungsstück oder an dem Gewebe die Leitfähigkeit der Leitungsdrähte durch chemische oder elektrochemische Behandlung oder durch Imprägnieren mit leitenden oder isolierenden Stoffen modifiziert ist.

7. Kleidungsstück gemäß den Ansprüchen 1 bis 6, **dadurch gekennzeichnet, dass** das Kleidungsstück aus mehreren Schichten übereinander gelagerten Gewebes (17, 18, 19) besteht, welche Leitungsdrähte enthalten können, insbesondere um die Schaltungen des Kleidungsstückes gegen unerwünschte elektromagnetische Effekte zu schützen und jeweils gegeneinander durch Drähte (20), welche sie durchdringen, gehalten werden.

8. Kleidungsstück gemäß den Ansprüchen 1 bis 7, **dadurch gekennzeichnet, dass** die elektrische Energie und/oder die Daten der Sensoren in dem Kleidungsstück durch einen Bus aus isolierten elastischen Leitungsdrähten (7) an denen die Sensoren realisiert sind, transportiert werden.

9. Kleidungsstück gemäß den Ansprüchen 1 bis 8, **dadurch gekennzeichnet, dass** die zum Funktionieren der Sensoren (3, 4, 5, 6) in dem Kleidungsstück erforderliche elektrische Energie durch induktive Kopplung ohne Kontakt zwischen einer äußeren Energiequelle und einer spezifischen Schaltung (8), welche in dem Kleidungsstück integriert ist, zur Verfügung gestellt ist.

10. Kleidungsstück gemäß den Ansprüchen 1 bis 9, **dadurch gekennzeichnet, dass** der Transport von durch die Sensoren des Kleidungsstückes erfassten Daten an eine nahe Sammelbasis (31) durch eine kontaktlose Verbindung realisiert wird, wie durch Kurzwellen oder Infrarotwellen oder durch induktive Kopplung.

11. Kleidungsstück gemäß den Ansprüchen 1 bis 10, **dadurch gekennzeichnet, dass** die durch die Sensoren gelieferten Daten über ein Telekommunikationsnetzwerk an einen für medizinisches Personal verwendbaren Server übertragbar sind.
